# EUROPÄISCHE PATENTSCHRIFT

(11) **EP 2 881 124 B1**
(45) Veröffentlichungstag und Bekanntmachung des Hinweises auf die Patenterteilung: **31.01.2018**
(21) Anmeldenummer: 14004052.8
(22) Anmeldetag: 02.12.2014
(51) Int. Cl.: A61L 2/07, B01L 1/02

(54) **Vorrichtung und Verfahren zur Behandlung von kleineren Gegenständen**
Device and method for treating small objects
Dispositif et procédé pour le traitement de petits objets

(30) Priorität: 09.12.2013 DE 102013020263
(43) Veröffentlichungstag der Anmeldung: 10.06.2015
(73) Patentinhaber: Atec Pharmatechnik Gmbh, 24966 Sörup (DE)
(72) Erfinder: Mumm, Hans-Werner, 24966 Sörup (DE)
(74) Vertreter: Thomas, Götz

(56) Entgegenhaltungen:
- EP-A1- 1 870 112
- EP-A1- 2 823 828
- EP-B1- 1 449 543
- EP-B1- 1 593 622
- WO-A1-00/61199
- WO-A1-00/74735
- WO-A1-03/070146
- CN-A- 102 688 718
- DE-A1- 2 739 169

## Beschreibung

Die vorliegende Erfindung betrifft eine Vorrichtung zur Behandlung von kleineren Gegenständen gemäß dem Oberbegriff des Anspruchs 1, sowie ein Verfahren zur Behandlung von kleineren Gegenständen gemäß dem Oberbegriff des Anspruchs 12.

Eine Vorrichtung der eingangs genannten Art ist zum Beispiel aus der EP 1 449 543 B des Anmelders bekannt, während Behälter der eingangs genannten Art unter anderem in der EP 1 510 227 B1 des Anmelders beschrieben sind. Die EP 1 169 067 B1 offenbart eine ähnliche Vorrichtung und einen ähnlichen Behälter. Solche Vorrichtungen und Behälter werden oft in Reinraumumgebungen eingesetzt, um ein aus Spritzenteilen, Verschlussstopfen aus Gummi oder Kunststoff für Infusionsflaschen oder Vials und dergleichen bestehendes Gut in den Behältern zu reinigen, zu sterilisieren und ggf. zu silikonisieren. Zur Behandlung des Guts wird dieses zuerst bei nach oben weisender Befüll- und Entleer-Öffnung in den Behälter gefüllt. Anschließend wird der mit dem Gut befüllte Behälter an einer schwenkbaren Halterung der Vorrichtung aufgehängt. Die Halterung weist mindestens zwei Medienanschlüsse auf, durch die während der Behandlung ein oder mehrere flüssige oder dampfförmige Behandlungs- oder Prozess-Medien, wie beispielsweise heißes Wasser oder Heißdampf, unter Verschwenken des Behälters durch diesen hindurchgeleitet werden können, um das Gut im Behälter der gewünschten Behandlung zu unterziehen und gleichzeitig in Bewegung zu versetzen. Nach der Behandlung können die Medienanschlüsse gelöst und der Behälter abgenommen werden, um ihn zum Beispiel zu einer Übergabestation zu transportieren und dort in umgedrehtem Zustand anzudocken, so dass das behandelte Gut von selbst durch die nach unten weisende Befüll- und Entleer-Öffnung in die Übergabestation fällt. Vor der Befüll- und Entleer-Öffnung des Behälters aus der EP 1 510 227 B1 ist ein Beta-Teil eines Rapid-Transfer-Ports angebracht, der gewährleistet, dass das Gut bei der Übergabe nicht kontaminiert werden kann.

Ein Rapid-Transfer-Port, der abgekürzt auch RTP genannt wird, umfasst einen an der Übergabestation vorgesehenen so genannten Alpha-Teil mit einer ersten Verschlussklappe, die normalerweise dichtend mit einer Deckelaufnahme des Alpha-Teils verriegelt ist, sowie einen am Behälter vorgesehenen so genannten Beta-Teil mit einer zweiten Verschlussklappe, die in geschlossenem Zustand mit einem umgebenden Flansch des Beta-Teils verriegelt ist und dabei gegen eine umlaufende Dichtung im Flansch angepresst wird. Beim Andocken des Beta-Teils an den Alpha-Teil werden die beiden Verschlussklappen miteinander verbunden, wobei die nach außen weisenden Oberflächen dichtend eingeschlossen werden. Wenn die Verschlussklappen dann gemeinsam ins Innere der Übergabestation hinein geöffnet werden, kann das zwischen den Klappen eingeschlossene Luftvolumen nicht mit dem Inneren der Übergabestation in Kontakt treten. Nach der Übergabe werden die Verschlussklappen mit dem eingeschlossenen Luftvolumen wieder gemeinsam verschlossen, so dass das Luftvolumen auch dabei nicht mit dem Inneren der Übergabestation in Kontakt treten kann.

Bei dem aus der EP 1 510 227 B1 bekannten Behälter werden die Behandlungsmedien bei der Behandlung durch zwei von der Befüll- und Entleer-Öffnung getrennte, mit den Medienanschlüssen der Halterung verbindbare Medienanschlüsse in den Behälter zu bzw. aus diesem abgeführt. Dies hat den Nachteil, dass der Beta-Teil des Rapid-Transfer-Ports und die Dichtung des Beta-Teils bei der Behandlung der Gegenstände nicht mitsterilisiert werden können, da der Beta-Teil bei der Behandlung nicht dem Heißdampf ausgesetzt ist. Entsprechendes gilt auch für einen in der WO 00/74735 A1 offenbarten Behälter, bei dem die Befüll- und Entleer-Öffnung mit einem Y-Anschluss versehen ist, der zudem relativ aufwändig ist und die Bauhöhe des Behälters erheblich vergrößert.

Um bei der Behandlung auch den Beta-Teil des Rapid-Transfer-Ports zu sterilisieren, wurde in der EP 1 593 622 B1 bereits vorgeschlagen, einen vom Behandlungsbehälter getrennten Transportbehälter zu verwenden, bei dem die Befüllöffnung und die Entleeröffnung an entgegengesetzten Enden des Behälters angeordnet sind, wobei die Entleeröffnung mit einem Rapid-Transfer-Port versehen ist, der einen Medienanschluss für ein Behandlungsmedium umfasst. Bei dieser Lösung werden jedoch statt eines Behälters zwei Behälter benötigt, was höhere Kosten verursacht. Nach der Behandlung des Guts muss außerdem der Transportbehälter am unteren Ende des Behandlungsbehälters angedockt werden, um diesen in den Transportbehälter hinein zu entleeren, was eine sehr große Raumhöhe und einen zusätzlichen Zeitaufwand erforderlich macht. Aufgrund der Trennung von Befüll- und Entleer-Öffnung muss der Transportbehälter darüber hinaus mit zwei größeren Öffnungen sowie den dazugehörigen Verschlussvorrichtungen versehen werden, wodurch der Herstellungsaufwand größer als bei einem Behälter mit einer einzigen Befüll- und Entleer-Öffnung ist.

Weitere ähnliche Behandlungsvorrichtungen sind in der WO 00/61199 A1 und in der EP 1 870 112 A1 offenbart, während weitere ähnliche Behandlungs- und/oder Transportbehälter in der EP 2 823 828 A1 und der DE 27 39 169 A1 beschrieben sind.

Ausgehend hiervon liegt der Erfindung die Aufgabe zugrunde, eine Vorrichtung und ein Verfahren der eingangs genannten Art dahingehend zu verbessern, dass zur Behandlung und zum Transport ein einziger Behälter ausreicht und dass die einzige Befüll- und Entleer-Öffnung sowie der davor angebrachte Beta-Teil des Rapid-Transfer-Ports einschließlich seiner Dichtung bei der Behandlung sterilisierbar ist.

Zur Lösung dieser Aufgabe werden bei der erfindungsgemäßen Vorrichtung die Merkmale im Anspruch 1 vorgeschlagen, während das erfindungsgemäße Verfahren durch die Merkmale im Anspruch 12 gekennzeichnet ist.

Durch die erfindungsgemäßen Maßnahmen können die einzige Befüll- und Entleer-Öffnung sowie der davor angebrachte Rapid-Transfer-Port und dessen Dichtung während der Behandlung der Gegenstände im Behälter mittels Heißdampf sterilisiert werden, der durch den Rapid-Transfer-Port hindurch in oder aus dem Behälter geleitet wird. Dadurch kann auf eine separate Sterilisierung dieser Komponenten verzichtet werden, ohne die Gefahr, dass die Gegenstände beim Befüllen oder Entleeren des Behälters mit unsterilen Komponenten in Kontakt treten können.

Nachfolgend wird zur Vereinfachung an Stelle des Begriffs "Beta-Teil des Rapid-Transfer-Ports" nur der Begriff "Rapid-Transfer-Port" oder "RTP" verwendet. Mit diesem vereinfachten Begriff ist jedoch immer der Beta-Teil des Rapid-Transfer-Ports gemeint.

Durch die mit dem Rapid-Transfer-Port bzw. dessen Beta-Teil versehene Befüll- und Entleer-Öffnung des Behälters und die an der Halterung vorgesehene Andock-, Öffnungs- und Schließ-Einrichtung zum Andocken an den Rapid-Transfer-Port und zum Öffnen und Schließen des Verschlussdeckels kann weiter einer der zur Zufuhr von Behandlungsmedium in den Behälter und/oder zur Abfuhr von Behandlungsmedium aus dem Behälter erforderlichen Medienanschlüsse in die Andock-, Öffnungs- und Schließ-Einrichtung integriert werden.

Bei dem Behälter kann zudem durch die erfindungsgemäßen Maßnahmen auf einen zweiten Medienanschluss neben der mit dem Rapid-Transfer-Port versehenen Befüll- und Entleer-Öffnung verzichtet und der Behälter trotzdem sowohl zur Behandlung und zum Transport des Guts eingesetzt werden, so dass kein weiterer Behälter nötig ist. Zusätzlich zur Befüll- und Entleer-Öffnung ist somit nur ein einziger Medienanschluss notwendig, der vorteilhaft an dem zur Befüll- und Entleer-Öffnung entgegengesetzten Ende des Behälters angeordnet ist.

Bei der Andock-, Öffnungs- und Schließ-Einrichtung handelt es sich vorzugsweise um eine Einrichtung, die beim Andocken an den Rapid-Transfer-Port des an der Halterung angebrachten Behälters selbsttätig gegenüber dem Rapid-Transfer-Port ausgerichtet wird und dann automatisch am Rapid-Transfer-Port andockt sowie mit dem Verschlussdeckel in Eingriff tritt und diesen öffnet. In diesem Zustand kommuniziert der in die Andock-, Öffnungs- und Schließ-Einrichtung integrierte Medienanschluss durch den Rapid-Transfer-Port hindurch mit dem Inneren des Behälters, so dass der Rapid-Transfer-Port beim Hindurchleiten von Dampf mit sterilisiert wird. Nach der Behandlung schließt die Andock-, Öffnungs- und Schließ-Einrichtung den Verschlussdeckel wieder automatisch und rückt aus dem Eingriff mit dem Verschlussdeckel aus, so dass der Behälter abgedockt wird und von der Halterung abgenommen werden kann.

Gemäß einer bevorzugten Ausgestaltung der Erfindung umfasst die Vorrichtung Mittel zum selbsttätigen Ausrichten der Andock-, Öffnungs- und Schließ-Einrichtung in einer definierten Lagebeziehung zu dem vor der Befüll- und Entleer-Öffnung angeordneten Rapid-Transfer-Port, so dass die Andock-, Öffnungs- und Schließ-Einrichtung Einrichtung im Zuge der Anbringung des Behälters an der schwenkbaren Halterung ohne zusätzliche Maßnahmen automatisch so ausgerichtet wird, dass sie beim Andocken gas- und flüssigkeitsdicht mit dem Rapid-Transfer-Port des Behälters verbunden wird.

Vorteilhaft umfassen die Mittel zum selbsttätigen Ausrichten paarweise zusammenwirkende Ausrichtelemente, die starr mit dem Behälter bzw. starr mit der Andock-, Öffnungs- und Schließ-Einrichtung verbunden sind. Auf diese Weise sorgt eine definierte Lagebeziehung zwischen den zusammenwirkenden Ausrichtelementen auch für eine definierte Lagebeziehung zwischen dem Rapid-Transfer-Port einerseits und der mit diesem zusammenwirkenden Andock-, Öffnungs- und Schließ-Einrichtung andererseits.

Um Toleranzen bei der Behälterfertigung Rechnung zu tragen, ist sind die Andock-, Öffnungs- und Schließ-Einrichtung sowie die Ausrichtelemente der Andock-, Öffnungs- und Schließ-Einrichtung vorteilhaft flexibel an der Halterung montiert, so dass sie in Bezug zur Halterung etwas beweglich sind, vorzugsweise in allen Raumrichtungen bzw. mit mehreren Freiheitsgraden, so dass sie sich an Lageabweichungen der Ausrichtelemente des Behälters anpassen können.

Um nach dem Andocken der Andock-, Öffnungs- und Schließ-Einrichtung an den Behälter ein Entweichen der durch den geöffneten Rapid-Transfer-Port strömenden Behandlungsmedien zu verhindern, umfasst die Andock-, Öffnungs- und Schließ-Einrichtung vorzugsweise eine nach unten zu offene Haube, in die der Medienanschluss mündet, sowie Dichtmittel zum Abdichten eines unteren Randes der Haube gegenüber dem Rapid-Transfer-Port oder dem Behälter.

Gemäß einer weiteren vorteilhaften Ausgestaltung der Erfindung umfassen die Dichtmittel in einer Nut des unteren Randes der Haube angeordnete ringförmige Dichtung, die sich durch Zufuhr von Druckfluid zwischen den Grund der Nut und die Dichtung gegen eine gegenüberliegende Stirnfläche des Rapid-Transfer-Ports oder des Behälters anpressen lässt. Auf diese Weise kann selbst ein verhältnismäßig breiter Spalt zwischen den gegenüberliegenden Stirnflächen am unteren Rand der Haube und am Rapid-Transfer-Port oder am Behälter abgedichtet werden.

Zum Öffnen des Verschlusselements des Rapid-Transfer-Ports ist die Andock-, Öffnungs- und Schließ-Einrichtung vorteilhaft mit einem Eingriffswerkzeug sowie einen ersten und einen zweiten Werkzeugantrieb zum Verfahren und Drehen des Eingriffswerkzeugs ausgestattet, um das letztere zuerst durch eine Linear- oder Schwenkbewegung an das Verschlusselement anzunähern und dann durch eine Drehung mit dem Verschlusselement in Eingriff zu bringen. Die Drehung, mit der das Eingriffswerkzeug mit dem Verschlusselement in Eingriff gebracht wird, kann zugleich genutzt werden, um das Verschlusselement des Rapid-Transfer-Ports zu entriegeln, während die Linear- oder Schwenkbewegung auch genutzt werden kann, um das Verschlusselement des Rapid-Transfer-Ports aus seinem Sitz heraus zu heben.

Um zu verhindern, dass flüssige Behandlungsmedien und/oder Verunreinigungen in eine nach oben offene Vertiefung im Verschlusselement des Rapid-Transfer-Ports eindringen und dort eine ordnungsgemäße Sterilisation des Rapid-Transfer-Ports und der Dichtung in Frage stellen, umfasst die Andock-, Öffnungs- und Schließ-Einrichtung vorteilhaft eine zusammen mit dem Eingriffswerkzeug bewegliche Scheibe oder Platte, welche die Vertiefung dicht verschließt, wenn sich das Eingriffswerkzeug im Eingriff mit dem Verschlusselement befindet. Die Scheibe oder Platte ist an ihrer Unterseite vorteilhaft mit einer ringförmigen Nut und einer in die Nut eingesetzte ringförmigen Dichtung versehen, die gegen die Oberseite eines Randes der Vertiefung angepresst wird, wenn sich das Eingriffswerkzeug im Eingriff mit dem Verschlusselement befindet.

Eine bevorzugte Ausgestaltung des erfindungsgemäßen Behälters sieht vor, dann in der Nähe der Befüll- und Entleer-Öffnung und des Rapid-Transfer-Ports Ausrichtelemente zum Zusammenwirken mit komplementären Ausrichtelementen der Andock-, Öffnungs- und Schließ-Einrichtung der Behandlungsvorrichtung vorgesehen sind, um das obere Ende des Behälter und die Andock-, Öffnungs- und Schließ-Einrichtung in Bezug zueinander so auszurichten, dass die Andock-, Öffnungs- und Schließ-Einrichtung anschließend gas- und flüssigkeitsdicht andocken kann.

Bei dem erfindungsgemäßen Verfahren wird nach der Behandlung der Rapid-Transfer-Port bzw. ein Verschlusselement desselben von der Andock-, Öffnungs- und Schließ-Einrichtung selbsttätig, d.h. ohne manuellen Eingriff, wieder geschlossen. Nachdem die Andock-, Öffnungs- und Schließ-Einrichtung vom Behälter gelöst worden ist, kann der Behälter von der Halterung abgenommen und zu der Übergabestation transportiert werden. Dort kann der Behälter umgedreht werden, um den Rapid-Transfer-Port an die Übergabestation anzudocken und zu öffnen, woraufhin der Behälter ohne die Gefahr einer Kontamination des Guts durch die nach unten weisende Befüll- und Entleer-Öffnung in die Übergabestation entleert werden kann.

Im Folgenden wird die Erfindung anhand eines in der Zeichnung dargestellten Ausführungsbeispiels näher beschrieben.
Fig. 1 zeigt eine perspektivische Ansicht einer erfindungsgemäßen Vorrichtung zur Reinigung und Sterilisation von pharmazeutischen Verschlüssen mit einem abnehmbaren und schwenkbaren Behandlungs- und Transportbehälter;
Fig. 2 zeigt eine Seitenansicht der Vorrichtung und des Behälters;
Fig. 3 zeigt eine Vorderseitenansicht eines Teils der Vorrichtung und des Behälters;
Fig. 4 zeigt eine vergrößerte perspektivische Ansicht einer Andock-, Öffnungs- und Schließ-Einrichtung der Vorrichtung und eines Teils des Behälters;
Fig. 5 zeigt eine andere teilweise geschnittene perspektivische Ansicht der Andock-, Öffnungs- und Schließ-Einrichtung der Vorrichtung und eines Teils des Behälters;
Fig. 6 zeigt weitere teilweise geschnittene perspektivische Ansicht der Andock-, Öffnungs- und Schließ-Einrichtung der Vorrichtung ohne den Behälter in einer abgesenkten Stellung eines Eingriffswerkzeugs;
Fig. 7 zeigt eine Ansicht entsprechend Fig. 6, jedoch in einer angehobenen Stellung des Eingriffswerkzeugs;
Fig. 8 zeigt eine Längsschnittansicht der Andock-, Öffnungs- und Schließ-Einrichtung und eines Teils des Behälters in der abgesenkten Stellung des Eingriffswerkzeugs um im Eingriff mit einem Rapid-Transfer-Port;
Fig. 9 zeigt eine perspektivische Ansicht der Andock-, Öffnungs- und Schließ-Einrichtung und eines Teils des Behälters bei der Annäherung des Behälters;
Fig. 10 zeigt eine Ansicht entsprechend Fig. 9, jedoch nach einer weiteren Annäherung des Behälters;
Fig. 11 zeigt eine Ansicht entsprechend Fig. 10, jedoch nach der Ausrichtung des Behälters in Bezug zur Andock-, Öffnungs- und Schließ-Einrichtung und nach dem Absenken des Eingriffswerkzeugs;
Fig. 12 zeigt eine Ansicht entsprechend Fig. 11, jedoch nach dem Öffnen des Rapid-Transfer-Ports.

Die in der Zeichnung dargestellte Vorrichtung 10 dient zur Reinigung, Sterilisation und ggf. zur Silikonisierung von Verschlüssen für pharmazeutische Verpackungsbehälter, die zu ihrer Behandlung in einen Behandlungs- und Transportbehälter 12 eingebracht und mit Hilfe der Vorrichtung 10 im Behälter 12 gereinigt, sterilisiert und ggf. silikonisiert werden, bevor sie im Behälter 12 zu einer Übergabestation (nicht dargestellt) einer Verpackungsmaschine transportiert werden, um sie dort nach einem Ankoppeln des Behälters 12 in sterilem Zustand zu entnehmen und mittels der Verpackungsmaschine einzeln auf den zuvor gefüllten pharmazeutischen Verpackungsbehältern anzubringen.

Wegen der hohen Anforderungen an die Reinheit erfolgen sowohl die Behandlung der Verschlüsse mit Hilfe der Reinigungs- und Sterilisationsvorrichtung 10 und der Transport des Behälters 12 mit den gereinigten und sterilisierten Verschlüssen sowie das Ankoppeln an die Übergabestation innerhalb eines Reinraums 14.

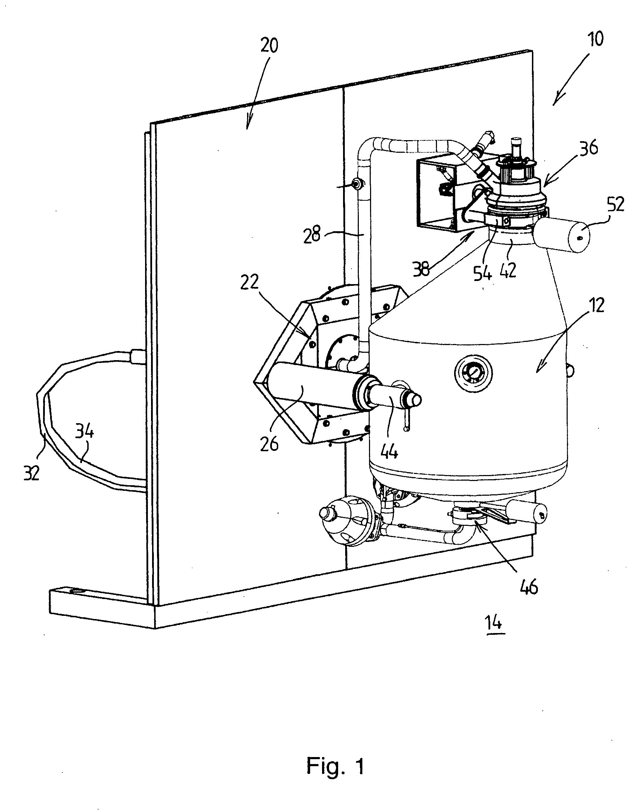

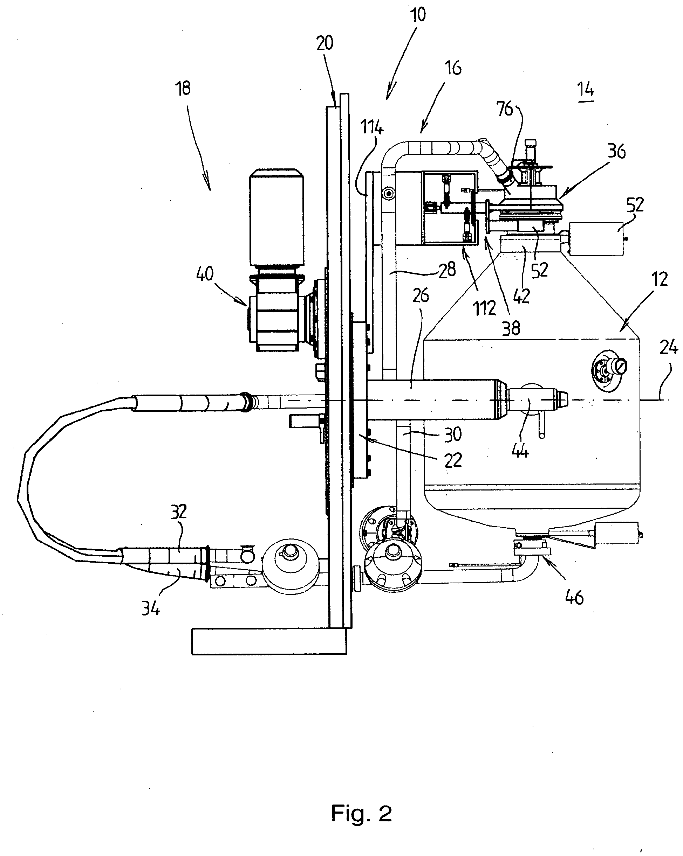

Wie am besten in Fig. 1 und 2 dargestellt, umfasst die Vorrichtung 10 einen reinraumseitigen Behälteraufnahmeteil 16 und einen hinter einer Wand 20 außerhalb des Reinraums 14 angeordneten Maschinenteil 18, der unter anderem zur Bereitstellung und Aufbereitung von Behandlungsmedien, wie Heiß- und Kaltwasser, Trocknungsluft, Dampf zur Sterilisierung oder andere flüssige oder gasförmige Behandlungsmedien dient.

Der Behälteraufnahmeteil 16 umfasst eine Halterung 22 zur lösbaren Befestigung des Behandlungs- und Transportbehälters 12, die sich motorisch um eine horizontale Schwenkachse 24 schwenken lässt und zwei in den Reinraum 14 überstehende parallele Tragdorne 26 für den Behälter 12 aufweist. Der Behälteraufnahmeteil 16 umfasst weiter zwei Leitungsabschnitte 28, 30 einer Zufuhr- bzw. Abfuhrleitung 32, 34, die sich durch die Halterung 22 und die Wand 20 hindurch in den Maschinenteil 18 erstrecken und zusammen mit der Halterung 22 um die Schwenkachse 24 schwenkbar sind. Durch die Zufuhr- bzw. Abfuhrleitung 32, 34 kann wahlweise ein Behandlungsmedium, wie Heiß- und Kaltwasser, ein Wasser-Luft-Gemisch, Trocknungsluft, Dampf oder ein anderes flüssiges oder gasförmiges Medium aus dem Maschinenteil 18 in den von der Halterung 22 aufgenommenen Behälter 12 zugeführt bzw. das aus dem Behälter 12 verdrängte Medium in den Maschinenteil 18 abgeführt werden. Außerdem umfasst der Behälteraufnahmeteil 16 eine gegenüber vom oberen Ende des Behälters 12 angebrachte automatische Andock-, Öffnungs- und Schließ-Einrichtung 36, sowie Ausrichtmittel 38 zum selbsttätigen Ausrichten der Andock-, Öffnungs- und Schließ-Einrichtung 36 in einer definierten Lagebeziehung zum oberen Ende des Behälters 12.

Der Maschinenteil 18 kann u.a. einen Heißwasserbereiter, einen Drucklufterzeuger und einen Dampferzeuger umfassen, die über die Zufuhr- bzw. Abfuhrleitung 32, 34 mit dem an der Halterung 22 angebrachten Behälter 12 verbindbar sind. Der Maschinenteil 18 umfasst weiter einen Schwenkantrieb 40 zum motorischen Verschwenken der Halterung 22 und des daran angebrachten Behälters 12 während der Behandlung. Eine ausführlichere Beschreibung des Behandlungsteils 16 und des Maschinenteils 16 findet sich in der eingangs genannten EP 1 449 543 B1, auf die in dieser Hinsicht Bezug genommen wird.

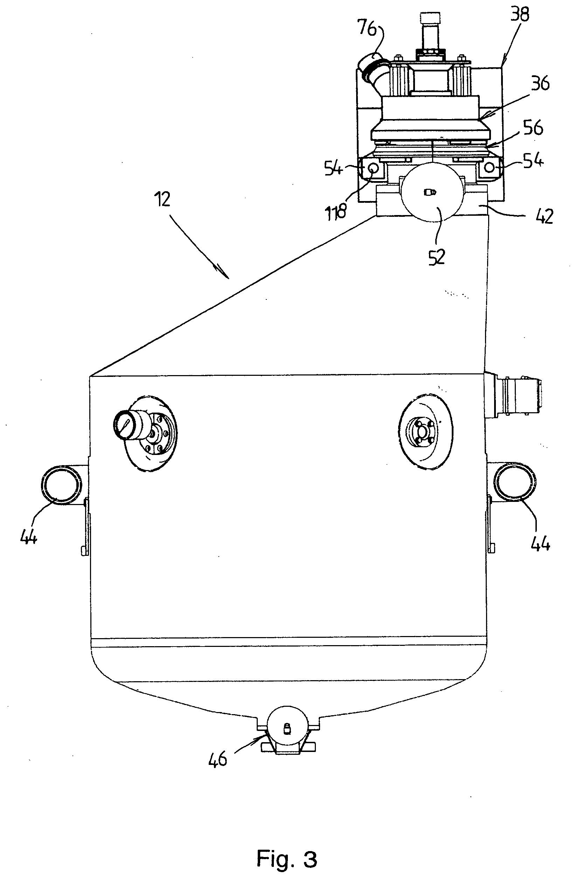

Der in Fig. 3 in aufrechter Stellung dargestellte Behälter 12 weist einen im Wesentlichen zylindrischen Unterteil mit einem konvex nach unten gewölbten Boden und einen nach oben zu verjüngten Oberteil mit einem verengten Halsteil 42 auf. Der Unterteil ist mit zwei diametral entgegengesetzten seitlichen Auslegern versehen, die zylindrische Aufnahmebuchsen 44 umgeben. Die Aufnahmebuchsen 44 sind bei aufrechtem Behälter 12 parallel und horizontal ausgerichtet und dienen zur Aufnahme der freien Enden der Tragdorne 26. Am tiefsten Punkt des Bodens befindet sich ein durch ein Klappenventil verschließbarer Medienanschluss 46, durch den eines der Behandlungsmedien aus dem Leitungsabschnitt 30 in den Behälter 12 zugeführt oder aus diesem abgeführt werden kann.

Der Halsteil 42 des Behälters 12 umgibt eine Befüll- und Entleer-Öffnung 48 mit größerem Öffnungsquerschnitt, durch welche die zu behandelnden Verschlüsse bei aufrechtem Behälter 12 in diesen eingefüllt werden und durch welche die Verschlüsse nach der Behandlung aufgrund ihrer Schwerkraft ausgetragen werden, nachdem der Behälter 12 zuvor umgedreht und an die Übergabestation angedockt worden ist. Neben dem Medienanschluss 46 und der Befüll- und Entleer-Öffnung 48 weist der Behälter 12 keinen weiteren Medienanschluss auf.

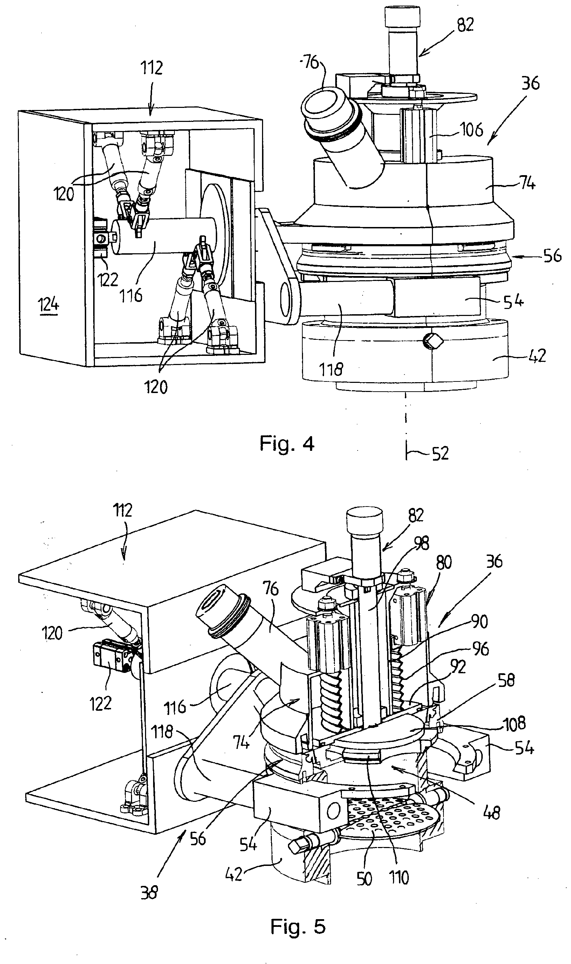

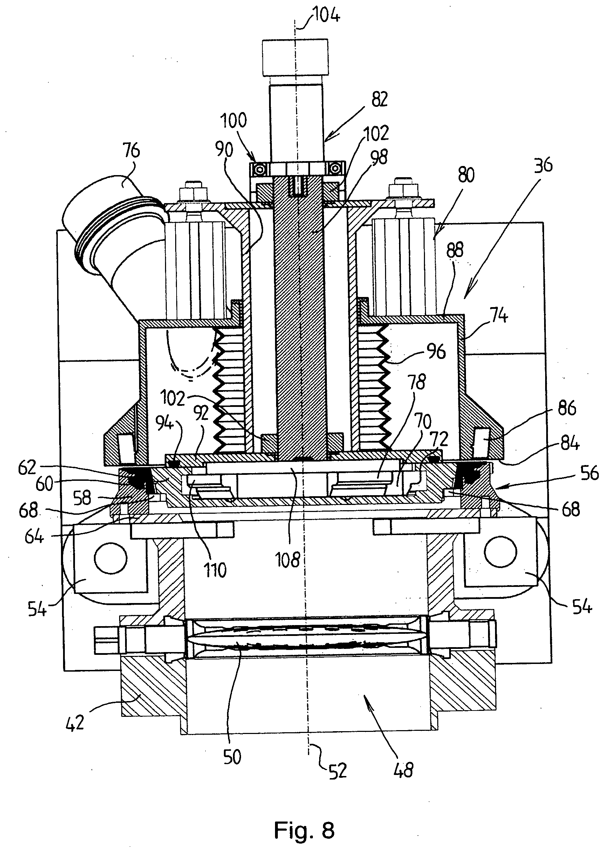

Wie am besten in Fig. 5 und 8 dargestellt, ist die Befüll- und Entleer-Öffnung 48 innerhalb des Halsteils 42 mit einer Lochklappe 50 versehen, die während der Behandlung der Verschlüsse senkrecht zu einer Längsmittelachse 52 des Halsteils 42 und der Öffnung 48 ausgerichtet ist und verhindert, dass Verschlüsse vom Behandlungsmedium durch die Öffnung 48 ausgetragen werden. Zum Entleeren des Behälters 12 kann die Lochklappe 50 mittels eines Drehantriebs 52 um 90 Grad um ihre Drehachse gedreht werden.

Weiter weist der Behälter 12 beiderseits des Halsteils 42 zwei zu den Aufnahmebuchsen 44 parallele Ausrichtbuchsen 54 mit konischen Aufnahmeöffnungen auf, mit denen der Halsteil 42 bei der Anbringung des Behälters 12 an der Halterung 22 in Bezug zur Andock-, Öffnungs- und Schließ-Einrichtung 36 in einer definierten Lagebeziehung ausgerichtet wird. Die Längsmittelachsen der konischen Aufnahmeöffnungen der Ausrichtbuchsen 54 sind parallel zu den Längsmittelachsen der Aufnahmebuchsen 44 ausgerichtet.

Wie am besten in den Figuren 5, 8 und 9 dargestellt, ist der Behälter 12 mit einem vor der Befüll- und Entleer-Öffnung 48 am oberen Ende des Halsteils 42 angebrachten Beta-Teil 56 eines Rapid-Transfer-Ports (nachfolgend verkürzt als Rapid-Transfer-Port 56 bezeichnet) versehen, der in geschlossenem Zustand einen sterilen Einschluss der Verschlüsse im Behälter 12 gewährleistet. Der Rapid-Transfer-Port 56 besteht im Wesentlichen aus einem Ringflansch 58, einer in den Ringflansch 58 eingesetzten Ringdichtung 60 und einem lösbaren Verschlussdeckel 62.

Der Ringflansch 58 ist mit einem Schraubflansch 64 am freien Ende des Halsteils 42 verschraubt. Der Verschlussdeckel 62 weist an seiner Unterseite radial nach außen überstehende Vorsprünge 66 auf, die sich von oben her durch entsprechende Aussparungen in einem nach innen überstehenden Bund des Ringflanschs 58 hindurchführen lassen und durch flache Schrägen an den Unterseiten des Bundes nach unten gedrückt werden, wenn der Verschlussdeckel 62 in Bezug zum Ringflansch 58 um die Längsmittelachse 52 gedreht wird und die Vorsprünge über die Schrägen gleiten. Dadurch wird der Verschlussdeckel 62 nach unten gezogen und mit einer konischen Umfangsfläche gegen eine komplementäre innere Umfangsfläche der Ringdichtung 60 angepresst, wodurch der Rapid-Transfer-Port 56 dicht verschlossen wird. An seiner Oberseite weist der Verschlussdeckel 62 eine nach oben offene Vertiefung 70 und einen am oberen Ende der Vertiefung 70 nach innen überstehenden Bund 72 auf, der ebenso wie der Bund des Flanschs 58 mit Aussparungen 73 versehen ist.

Zum Entleeren des Behälters 12 an der Übergabestation kann der Rapid-Transfer-Port 56 in bekannte Weise geöffnet werden. Außerdem kann der Rapid-Transfer-Port 56 auch nach der Anbringung des Behälters 12 an der Halterung 22 und dem Andocken an die Andock-, Öffnungs- und Schließ-Einrichtung 36 geöffnet werden, um während der Behandlung der Verschlüsse Behandlungsmedien durch die Befüll- und Entleer-Öffnung 48 und den geöffneten Rapid-Transfer-Port 56 in den Behälter 12 zu bzw. aus dem Behälter 12 abzuführen.

Wie am besten in den Figuren 5 bis 8 dargestellt, umfasst die zum Andocken des Behälters 12 sowie zum Öffnen und Schließen des Rapid-Transfer-Ports 56 dienende Andock-, Öffnungs- und Schließ-Einrichtung 36 eine nach unten offene, ansonsten jedoch geschlossene zylindrische Haube 74, die sich von oben her dichtend auf den Schraub- oder Ringflansch 58 und die Dichtung 60 des Rapid-Transfer-Ports 56 aufsetzen lässt. In die Haube 74 mündet ein mit dem Leitungsabschnitt 28 verbundener Medienanschluss 76, durch den ein Behandlungsmedium aus dem Maschinenteil 18 in die Haube 74 und von dort durch den offenen Rapid-Transfer-Port 56 in den Behälter 12 zugeführt bzw. umgekehrt aus dem Behälter 12 durch den offenen Rapid-Transfer-Port 56 in die Haube 74 und von dort durch den Medienanschluss 76 und den Leitungsabschnitt 28 in den Maschinenteil 18 abgeführt werden kann.

Außerdem umfasst die Einrichtung 36 ein innerhalb der Haube 74 angeordnetes Eingriffswerkzeug 78, einen ersten Werkzeugantrieb 80 zum Anheben und Absenken des Eingriffswerkzeugs 78 in Bezug zur Haube 74, sowie einen zweiten Werkzeugantrieb 82 zum Drehen des Eingriffswerkzeugs 78 in Bezug zur Haube 74. Beide Antriebe 80, 82 sind oberhalb von der Haube 74 angeordnet. Das Eingriffswerkzeug 78 lässt sich mittels des ersten Werkzeugantriebs 80 in der Haube 74 auf und ab bewegen und mittels des zweiten Werkzeugantriebs 82 drehen, um es zum Öffnen des Rapid-Transfer-Ports 56 in die Vertiefung 70 des Verschlussdeckels 62 abzusenken, mit dem Verschlussdeckel 62 in Eingriff zu bringen, den Verschlussdeckel 62 zum Entriegeln zu drehen und ihn dann zum Abnehmen aus dem Ringflansch 60 heraus anzuheben bzw. um ihn zum Verschließen des Rapid-Transfer-Ports 56 wieder bis zur Anlage gegen die Dichtung 60 in den Flansch 58 abzusenken, ihn dann zum Verriegeln mit dem Flansch 58 mit entgegengesetzter Drehrichtung zu drehen und um anschließend das Eingriffswerkzeug 78 ebenfalls durch Drehen aus dem Eingriff mit dem Verschlussdeckel 62 auszurücken und es zuletzt wieder nach oben aus der Vertiefung 70 heraus zu heben.

Der ringförmige untere Rand der Haube 74 besitzt eine ebene Stirnfläche 84 und eine an der Stirnfläche 84 mündende, nach unten offene Nut 86, in die eine Dichtung (nicht dargestellt) eingesetzt ist. Zwischen den Grund der Nut 86 und die Dichtung kann Druckluft oder unter Druck stehender Dampf zugeführt werden, um die Dichtung dichtend gegen die gegenüberliegende obere ebene Stirnfläche des Flanschs 58 bzw. der Dichtung 60 des Rapid-Transfer-Ports 56 anzupressen und dadurch das Innere der Haube 74 flüssigkeits- und gasdicht gegen die Umgebung abzudichten. Der Innendurchmesser der Haube 74 ist geringfügig größer als der Außendurchmesser des Verschlussdeckels 62, so dass sich dieser nach dem Entriegeln innerhalb der Haube 74 nach oben bewegen lässt, um ihn aus seinem Sitz im Flansch 58 bzw. in der Dichtung 60 heraus zu heben und den Rapid-Transfer-Port 56 zu öffnen. An ihrem oberen Ende weist die Haube 74 eine Stirnwand 88 mit einer mittigen Durchtrittsöffnung auf. Durch die Durchtrittsöffnung erstreckt sich ein vertikales Rohr 90, das zusammen mit dem Eingriffswerkzeug 78 und dem Deckel 62 auf und ab beweglich ist, das sich jedoch nicht mit dem Eingriffswerkzeug 78 mitdreht. Das untere Ende des Rohrs 90 ist starr mit einer zur Stirnfläche 84 parallelen ebenen Scheibe 92 verbunden, deren Außendurchmesser größer als der Durchmesser der Vertiefung 70 des Verschlussdeckels 62 ist. Die Scheibe 92 ist an ihrer Unterseite mit einer nach unten offenen Nut und einer in die Nut eingesetzten Dichtung 94 versehen, die dichtend gegen die obere Stirnfläche des Verschlussdeckels 62 angepresst werden kann, um ein Eindringen von Behandlungsmedien und Schmutz aus dem Inneren der Haube 74 in die Vertiefung 70 zu verhindern, insbesondere auch ein Eindringen von Wasser, das eine ordnungsgemäße Sterilisation verhindern könnte. Innerhalb der Haube 74 ist das Rohr 90 von einem Faltenbalg 96 umgeben, dessen oberes Ende dicht mit der Stirnwand 88 und dessen unteres Ende dicht mit der Scheibe 92 verbunden ist.

Das Rohr 90 umgibt eine vertikale Abtriebswelle 98 des zweiten Werkzeugantriebs 82, der als elektrischer Drehantrieb auf einer starr mit dem oberen Ende des Rohrs 90 verbundenen Konsole 100 montiert ist. Die Welle 98 ist in zwei Axial- und Radiallagern 102 am oberen und unteren Ende des Rohrs 90 gelagert, erstreckt sich durch eine mittige Öffnung der Scheibe 92 und ist an ihrem unteren Ende starr mit dem Eingriffswerkzeug 78 verbunden, so dass dieses mittels des Antriebs 82 um eine vertikale Drehachse 104 in Bezug zum Rohr 90, zur Haube 74 und zur Scheibe 92 gedreht werden kann. Am oberen Ende ist das Rohr 90 durch eine Platte verschlossen, auf der sich das obere Lager 102 abstützt. Das untere Lager 102 stützt sich auf der Scheibe 92 ab.

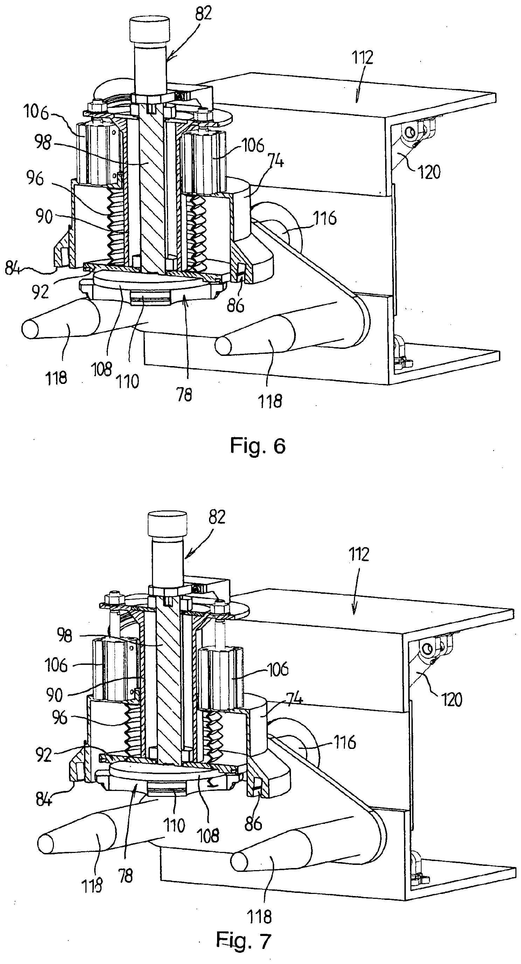

Das Rohr 90 lässt sich mittels des ersten Werkzeugantriebs 80 zusammen mit der Welle 98, dem Eingriffswerkzeug 78 und der Scheibe 92 innerhalb der Haube 74 anheben oder absenken, wobei sich der Faltenbalg 96 verkürzt oder verlängert, wie aus Fig. 6 und 7 ersichtlich ist. Der erste Werkzeugantrieb 80 umfasst zwei Hydraulik- oder Pneumatikzylinder 106, deren Zylinderrohre auf der Oberseite der Stirnwand 88 der Haube 74 abgestützt sind und deren nach oben ausfahrbare Kolbenstangen mit einem nach außen überstehenden Flansch am oberen Ende des Rohrs 90 verbunden sind.

Das drehfest mit dem unteren Ende der Welle 98 verbundene Eingriffswerkzeug 78 umfasst eine unterhalb von der Scheibe 92 angeordnete runde Platte 108, die in einer flachen Vertiefung an der Unterseite der Scheibe 92 gegen diese anliegt und in Bezug zur Scheibe 92 drehbar ist. Über die Unterseite der Platte 108 stehen vier Vorsprünge 110 radial nach außen über, die sich von oben her durch die Aussparungen in dem nach innen überstehenden Bund 72 des Verschlussdeckels 62 hindurchbewegen und dann durch Drehen des Eingriffswerkzeug 78 mittels des zweiten Werkzeugantriebs 82 um die Drehachse 104 unter den Bund bewegen 72 und dadurch mit dem Verschlussdeckel 62 axial unbeweglich in Eingriff bringen lassen. Ein ordnungsgemäßer Eingriff ist erreicht, die Vorsprünge 110 gegen die Ränder der Aussparungen des Bundes 72 anschlagen. Wenn in dieser Position das Eingriffswerkzeug 78 weiter gedreht wird, wird der Verschlussdeckel 62 von den Vorsprüngen 110 in Bezug zum Ringflansch 58 gedreht und dadurch entriegelt, d.h. aus seinem Sitz im Ringflansch 58 gelöst. Da das Eingriffswerkzeug 78 den Bund 72 untergreift, kann der entriegelte Verschlussdeckel 62 anschließend durch Ausfahren der Kolbenstangen der Zylinder 106 des Werkzeugsantriebs 80 zusammen mit dem Eingriffswerkzeug 78 angehoben werden, um den Rapid-Transfer-Port 56 zu öffnen.

Bei geöffnetem Verschlussdeckel 62 können Behandlungsmedien durch den Rapid-Transfer-Port 56 und an der Ringdichtung 60 vorbei in und aus dem Behälter 12 geleitet werden, so dass die Ringdichtung 60 durch den Kontakt mit Heißdampf sterilisiert werden kann.

Die Ausrichtmittel 38 stellen bei der Anbringung des Behälters 12 an der Halterung 22 selbsttätig sicher, dass der Halsteil 42 des Behälters 12 und die Andock-, Öffnungs- und Schließ-Einrichtung 36 in Bezug zueinander so ausgerichtet werden, dass zum einen die Längsmittelachse 52 der Befüll- und Entleer-Öffnung 48 und damit des Rapid-Transfer-Ports 56 mit der Drehachse 104 des zweiten Werkzeugantriebs 82 und damit mit der Mittelachse der Haube 74 und des Eingriffswerkzeugs 78 fluchtet, und dass zum anderen die untere Stirnfläche 84 der Haube 74 spaltfrei und planparallel mit der gegenüberliegenden oberen Stirnfläche des Rapid-Transfer-Ports 56 zu Anlage gebracht wird.

Wie am besten in den Figuren 4 bis 7 und 9 bis 12 dargestellt, umfassen die Ausrichtmittel 38 neben den Ausrichtbuchsen 54 am Halsteil 42 des Behälters 12 eine mittels eines Tragrahmens 114 (Fig. 2) starr an der Halterung 22 befestigte Konsole 112, eine in der Konsole 112 flexibel aufgehängte und starr mit der Haube 74 verbundene Achse 116 und zwei unterhalb von der Andock-, Öffnungs- und Schließ-Einrichtung 36 angeordnete, starr mit der Achse 116 und der Haube 74 verbundene Ausrichtdorne 118, deren Mittenabstand dem Mittenabstand der Ausrichtbuchsen 54 des Behälters 12 entspricht. Die Ausrichtdorne 118 sind parallel zur Achse 116 ausgerichtet und weisen konisch verjüngte freie Enden auf.

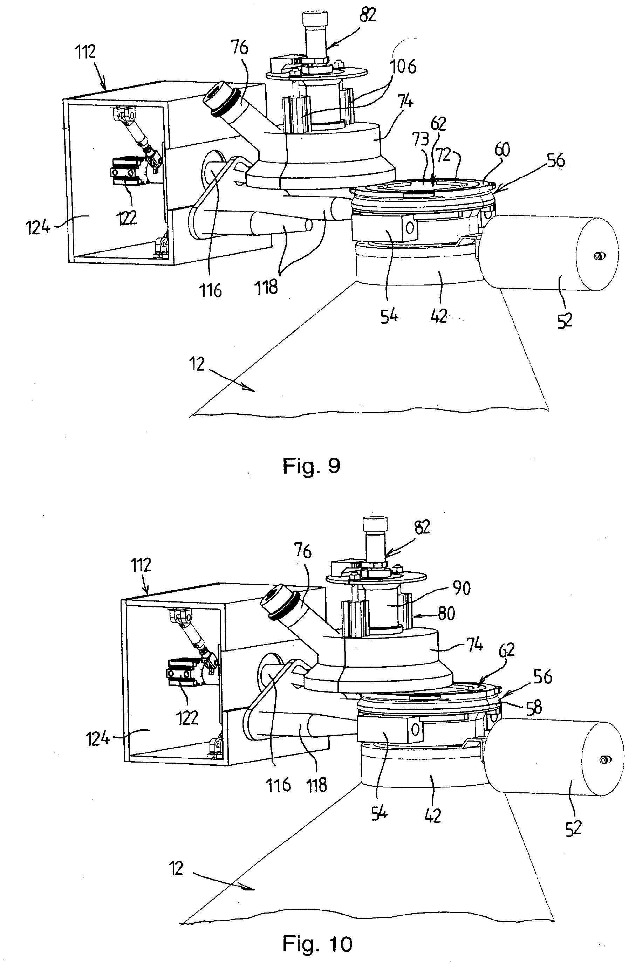

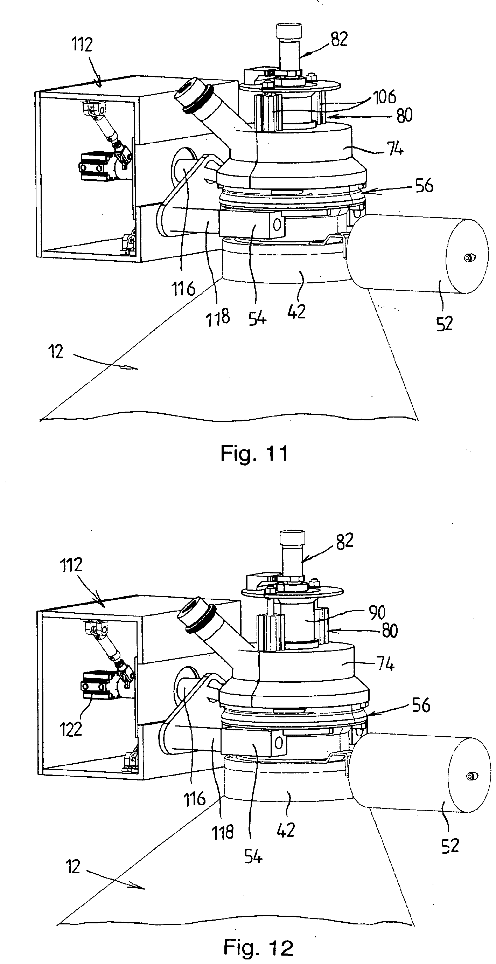

Wenn bei der Anbringung des Behälters 12 an der Halterung 22 die Aufnahmebuchsen 44 am Behälterunterteil auf die Tragdorne 26 der Halterung 22 aufgeschoben werden, werden die freien konischen Enden der Ausrichtdorne 118 in die komplementär geformten und bemessenen konischen Aufnahmeöffnungen der Ausrichtbuchsen 54 eingeführt, wie in Fig. 9 und 10 dargestellt. Durch die starre Verbindung der Ausrichtdorne 118 mit der Haube 74 der Andock-, Öffnungs- und Schließ-Einrichtung 36 kann sichergestellt werden, dass die Ausrichtdorne 118 sowohl zur Drehachse 104 der Abtriebswelle 98 als auch zur Stirnfläche 84 der Haube eine genau definierte Lagebeziehung aufweisen. Durch die starre Verbindung der Ausrichtbuchsen 54 mit dem Halsteil 42 des Behälters 12 und durch eine identische Lagebeziehung der Ausrichtbuchsen 54 in Bezug zur Längsmittelachse der Befüll- und Entleer-Öffnung 48 und in Bezug zur oberen Stirnfläche des Rapid-Transfer-Ports 56 kann weiter gewährleistet werden, dass die Längsmittelachse 52 der Befüll- und Entleer-Öffnung 48 genau mit der Drehachse 104 fluchtet und die gegenüberliegenden Stirnflächen spaltfrei und planparallel gegeneinander anliegen, wenn sich die Ausrichtdorne 118 vollständig innerhalb der Ausrichtbuchsen 54 befinden, wie in Fig. 11 und 12 dargestellt. Mit anderen Worten wird durch das Zusammenwirken der konischen Ausrichtdorne 118 und der konischen Aufnahmeöffnungen der Ausrichtbuchsen 54 eine selbsttätige Zentrierung oder Ausrichtung des Rapid-Transfer-Ports 56 in Bezug zur Andock-, Öffnungs- und Schließ-Einrichtung 36 unbewirkt.

Da es sich bei den Behältern 12 um Schweißbehälter handelt, bei denen von Behälter zu Behälter verschiedene Toleranzen zwischen den Aufnahmebuchsen 44 und den Ausrichtbuchsen 54 vorhanden sein können, lässt sich jedoch nicht sicherstellen, dass bei der Anbringung der Behälter 12 an der Halterung 22 die Ausrichtbuchsen 54 stets genau dieselbe Raumlage und Ausrichtung einnehmen. Da dies im Fall einer starren Befestigung der Andock-, Öffnungs- und Schließ-Einrichtung 36 an der Halterung 22 dazu führen könnte, dass die Ausrichtdorne 58 nicht genau in die Ausrichtbuchsen 54 eintreten können oder sich in den Ausrichtbuchsen 54 verspannen, ist die Andock-, Öffnungs- und Schließ-Einrichtung 36 zusammen mit den Ausrichtdornen 118 so an der Halterung 22 befestigt, dass sie in allen Raumrichtungen, das heißt mit mehreren Freiheitsgraden flexibel beweglich ist.

Zu diesem Zweck ist die die Andock-, Öffnungs- und Schließ-Einrichtung 36 tragende Achse 116 innerhalb der Konsole 112 an mehreren, in gleichen Winkelabständen um die Achse 116 herum angeordneten Pneumatik-Dämpfungszylindern 120 schwebend aufgehängt. Außerdem stützt sie sich mit ihrem von der Einrichtung 36 abgewandten Ende über einen weiteren Pneumatik-Dämpfungszylinder 122 gegen eine Rückwand 124 der Konsole 112 ab.

## Patentansprüche

1. Behandlungsvorrichtung (10) zur Reinigung und Sterilisation von kleineren Gegenständen für pharmazeutische Zwecke, mit einer motorisch schwenkbaren Halterung (22), einem an der Halterung (22) anbringbaren Behandlungs- und/oder Transportbehälter (12) zur Behandlung und/oder zum Transport der Gegenstände, der eine in einer Befüllstellung des Behälters (12) nach oben und in einer Entleerstellung des Behälters (12) nach unten weisende Befüll- und Entleer-Öffnung (48) für die Gegenstände und vor der Befüll- und Entleer-Öffnung (48) einen Rapid-Transfer-Port (56) mit einem Verschlussdeckel (62) aufweist, **dadurch gekennzeichnet, dass** an der Halterung (22) eine zusammen mit der Halterung (22) schwenkbare Andock-, Öffnungs- und Schließ-Einrichtung (36) angebracht ist, mit der sich nach dem Andocken am Rapid-Transfer-Port (56) der Verschlussdeckel (62) öffnen und schließen lässt, wobei die Andock-, Öffnungs- und Schließ-Einrichtung (36) ein Eingriffswerkzeug (78), sowie einen ersten und einen zweiten Werkzeugantrieb (80, 82) zum Verfahren und Drehen des Eingriffswerkzeugs (78) umfasst, und weiter einen Medienanschluss (76) aufweist, wobei sich das Eingriffswerkzeug (78) mit dem Verschlussdeckel (62) in Eingriff bringen lässt, um den Verschlussdeckel (62) zum Öffnen zu drehen und anzuheben und wobei der Medienanschluss (76) zur Zufuhr von Behandlungsmedium in den Behälter (12) und/oder zur Abfuhr von Behandlungsmedium aus dem Behälter (12) durch den geöffneten Rapid-Transfer-Port (56) dient.

2. Behandlungsvorrichtung nach Anspruch 1, **gekennzeichnet durch** Mittel (38) zum selbsttätigen Ausrichten der Andock-, Öffnungs- und Schließ-Einrichtung (36) in einer definierten Lagebeziehung zum Rapid-Transfer-Port (56).

3. Behandlungsvorrichtung nach Anspruch 2, **dadurch gekennzeichnet, dass** die Mittel (38) zum selbsttätigen Ausrichten paarweise zusammenwirkende Ausrichtelemente (54, 118) umfassen, die starr mit dem Behälter (12) bzw. mit der Andock-, Öffnungs- und Schließ-Einrichtung (36) verbunden sind.

4. Behandlungsvorrichtung nach einem der vorangehenden Ansprüche, **dadurch gekennzeichnet, dass** die Andock-, Öffnungs- und Schließ-Einrichtung (36) in Bezug zur Halterung (23) beweglich ist.

5. Behandlungsvorrichtung nach einem der vorangehenden Ansprüche, **dadurch gekennzeichnet, dass** die Andock-, Öffnungs- und Schließ-Einrichtung (36) eine nach unten zu offene Haube (74) und Mittel zum Abdichten eines unteren Randes der Haube (74) gegenüber dem Rapid-Transfer-Port (56) oder dem Behälter (12) umfasst.

6. Behandlungsvorrichtung nach Anspruch 5, **dadurch gekennzeichnet, dass** die Andock-, Öffnungs- und Schließ-Einrichtung (36) einen ersten Werkzeugantrieb (80) zum Anheben und Absenken des Eingriffswerkzeugs (78) in Bezug zur Haube (74) und einen zweiten Werkzeugantrieb (82) zum Drehen des Eingriffswerkzeugs (78) in Bezug zur Haube (74) umfassen.

7. Behandlungsvorrichtung nach Anspruch 6, **dadurch gekennzeichnet, dass** die Andock-, Öffnungs- und Schließ-Einrichtung (36) eine zusammen mit dem Eingriffswerkzeug (78) verfahrbare Scheibe (92) oder Platte zum dichten Verschließen einer Vertiefung (70) im Verschlusselement (62) des Rapid-Transfer-Ports (56) umfasst.

8. Behandlungsvorrichtung nach einem der vorangehenden Ansprüche, **dadurch gekennzeichnet, dass** der Behandlungs- und Transportbehälter (12) Mittel (44) zur lösbaren Anbringung an der schwenkbaren Halterung (22) der Behandlungsvorrichtung (10), eine in einer Befüllstellung des Behälters (12) nach oben und in einer Entleerstellung des Behälters (12) nach unten weisende einzige Befüll- und Entleer-Öffnung (48) für die Gegenstände und einen vor der Befüll- und Entleer-Öffnung (48) angebrachten Rapid-Transfer-Port (56) mit einem Verschlussdeckel (62) umfasst, der sich mittels einer an der Halterung (22) angebrachten, zusammen mit der Halterung (22) schwenkbaren Andock-, Öffnungs- und Schließ-Einrichtung (36) öffnen und schließen lässt, dass am Behälter (12) abgesehen von der Befüll- und Entleer-Öffnung (48) nur ein einziger Medienanschluss (46) vorgesehen ist und dass die Zufuhr und/oder Abfuhr von Behandlungsmedium aus der Behandlungsvorrichtung (10) zum einen durch den Medienanschluss (46) sowie zum anderen durch einen Medienanschluss (76) der an den Rapid-Transfer-Port (56) angedockten Andock-, Öffnungs- und Schließ-Einrichtung (36) und den geöffneten Rapid-Transfer-Port (56) erfolgt.

9. Behandlungsvorrichtung nach Anspruch 8, **dadurch gekennzeichnet, dass** der Medienanschluss (46) an einem zur Befüll- und Entleer-Öffnung (48) entgegengesetzten Ende des Behälters (12) angeordnet ist.

10. Behandlungsvorrichtung nach Anspruch 8 oder 9, **dadurch gekennzeichnet, dass** der Behälter (12) Ausrichtelemente (54) in der Nähe der Befüll- und Entleer-Öffnung (48) und des Rapid-Transfer-Ports (56) zum Zusammenwirken mit komplementären Ausrichtelementen (118) der Andock-, Öffnungs- und Schließ-Einrichtung (36) der Behandlungsvorrichtung (10) umfasst.

11. Behandlungsvorrichtung nach einem der Ansprüche 8 bis 10, **gekennzeichnet durch** eine in der Befüll- und Entleer-Öffnung (48) des Behälters (12) angeordnete schwenkbare Lochklappe (50) .

12. Verfahren zur Reinigung und Sterilisation sowie zum Transport von kleineren Gegenständen für pharmazeutische Zwecke, bei dem eine Mehrzahl der Gegenstände in einen Behandlungs- und Transportbehälter (12) mit einer einzigen Befüll- und Entleer-Öffnung (48) und einem vor der Befüll- und Entleer-Öffnung (48) angebrachten Rapid-Transfer-Port (56) eingebracht wird, bei dem der Behälter (12) anschließend lösbar an einer motorisch schwenkbaren Halterung (22) einer Behandlungsvorrichtung (10) angebracht wird, **dadurch gekennzeichnet, dass** nach der Anbringung des Behälters (12) an der Halterung (22) ein Verschlussdeckel (62) des Rapid-Transfer-Port (56) mittels einer zusammen mit der Halterung (22) schwenkbaren Andock-, Öffnungs- und Schließ-Einrichtung (36) geöffnet und mindestens ein Behandlungsmedium durch einen Medienanschluss (76) der Andock-, Öffnungs- und Schließ-Einrichtung (36) sowie durch den geöffneten Rapid-Transfer-Port (56) in den Behälter (12) zugeführt oder aus dem Behälter (12) abgeführt wird.

13. Verfahren nach Anspruch 12, **dadurch gekennzeichnet, dass** der Rapid-Transfer-Port (56) an einer Andock-, Öffnungs- und Schließ-Einrichtung (36) der Halterung (22) angedockt und von der Andock-, Öffnungs- und Schließ-Einrichtung (36) selbsttätig geöffnet und wieder geschlossen wird.

## Claims

1. Treatment device (10) for cleaning and sterilizing small objects for pharmaceutical purposes, with a motor-pivotable holder (22), a treatment and/or transport container (12) mountable on the holder (22) and used for treating and/or transporting the objects, which treatment and/or transport container (12) has a filling and emptying opening (48) for the objects, that points upwards in a filling position of the container (12) and downwards in an emptying position of the container (12), and, outside of the filling and emptying opening (48), a rapid transfer port (56) with a closure lid (62), **characterized in that** a docking, opening and closing mechanism (36), pivotable together with the holder (22), is mounted on the holder (22), with which mechanism the closure lid (62) can be opened and closed after the docking on the rapid transfer port (56), wherein the docking, opening and closing mechanism (36) has an engagement tool (78) and a first and a second tool drive (80, 82) for driving and turning the engagement tool (78), and it moreover has a medium connection (76), wherein the engagement tool (78) can be brought into engagement with the closure lid (62) in order to open the closure lid (62) by turning and lifting it, and wherein the medium connection (76) serves to deliver treatment medium into the container (12) and/or to remove treatment medium from the container (12) through the opened rapid transfer port (56).

2. Treatment device according to Claim 1, **characterized by** means (38) for automatic alignment of the docking, opening and closing mechanism (36) in a defined position with respect to the rapid transfer port (56).

3. Treatment device according to Claim 2, **characterized in that** the means (38) for automatic alignment comprise mutually interacting alignment elements (54, 118) that are rigidly connected to the container (12) and to the docking, opening and closing mechanism (36), respectively.

4. Treatment device according to one of the preceding claims, **characterized in that** the docking, opening and closing mechanism (36) is movable with respect to the holder (23).

5. Treatment device according to one of the preceding claims, **characterized in that** the docking, opening and closing mechanism (36) comprises a downwardly open hood (74) and means for sealing a lower edge of the hood (74) in relation to the rapid transfer port (56) or to the container (12).

6. Treatment device according to Claim 5, **characterized in that** the docking, opening and closing mechanism (36) comprises a first tool drive (80) for lifting and lowering the engagement tool (78) with respect to the hood (74) and a second tool drive (82) for turning the engagement tool (78) with respect to the hood (74).

7. Treatment device according to Claim 6, **characterized in that** the docking, opening and closing mechanism (36) comprises a disc (92) or plate which is movable together with the engagement tool (78) in order to sealingly close a depression (70) in the closure element (62) of the rapid transfer port (56).

8. Treatment device according to one of the preceding claims, **characterized in that** the treatment and transport container (12) comprises means (44) for releasable mounting on the pivotable holder (22) of the treatment device (10), a single filling and emptying opening (48) pointing upwards in a filling position of the container (12) and downwards in an emptying position of the container (12), and a rapid transfer port (56) mounted in front of the filling and emptying opening (48) and having a closure lid (62) which can be opened and closed by means of a docking, opening and closing mechanism (36) mounted on the holder (22) and pivotable together with the holder (22), **in that**, apart from the filling and emptying opening (48), only a single medium connection (46) is provided on the container (12), and **in that** the delivery and/or removal of treatment medium from the treatment device (10) is effected, on the one hand, through the medium connection (46) and, on the other hand, through a medium connection (76) of the docking, opening and closing mechanism (36) docked on the rapid transfer port (56), and the opened rapid transfer port (56).

9. Treatment device according to Claim 8, **characterized in that** the medium connection (46) is arranged at an end of the container (12) that is opposite from the filling and emptying opening (48) .

10. Treatment device according to Claim 8 or 9, **characterized in that** the container (12) comprises alignment elements (54) in the proximity of the filling and emptying opening (48) and of the rapid transfer port (56), for interaction with complementary alignment elements (118) of the docking, opening and closing mechanism (36) of the treatment device (10).

11. Treatment device according to one of Claims 8 to 10, **characterized by** a pivotable perforated flap (50) arranged in the filling and emptying opening (48) of the container (12).

12. Method for cleaning and sterilizing and for transporting small objects for pharmaceutical purposes, in which method a plurality of the objects are introduced into a treatment and/or transport container (12) having a single filling and emptying opening (48) and a rapid transfer port (56) mounted outside of the filling and emptying opening (48), and in which the container (12) is then mounted releasably on a motor-pivotable holder (22) of a treatment device (10), **characterized in that**, after the container (12) has been mounted on the holder (22), a closure lid (62) of the rapid transfer port (56) is opened by means of a docking, opening and closing mechanism (36) pivotable together with the holder (22), and at least one treatment medium is delivered to the container (12) or removed from the container (12) through a medium connection (76) of the docking, opening and closing mechanism (36) and through the opened rapid transfer port (56).

13. Method according to Claim 12, **characterized in that** the rapid transfer port (56) is docked on a docking, opening and closing mechanism (36) of the holder (22) and is automatically opened and closed again by the docking, opening and closing mechanism (36).

## Revendications

1. Dispositif de traitement (10) destiné au nettoyage et à la stérilisation d'objets relativement petits pour des utilisations pharmaceutiques, avec un support (22) pouvant pivoter de façon motorisée, un récipient de traitement et/ou de transport (12) pouvant être mis en place sur le support (22) en vue du traitement et/ou du transport des objets, qui présente une ouverture de remplissage et de vidage (48) pour les objets dirigée vers le haut dans une position de remplissage du récipient (12) et vers le bas dans une position de vidage du récipient (12) et, devant l'ouverture de remplissage et de vidage (48), un orifice de transfert rapide (56) avec un couvercle de fermeture (62), **caractérisé en ce que**, sur le support (22), il est mis en place un système d'arrimage, d'ouverture et de fermeture (36), pouvant pivoter conjointement avec le support (22), avec lequel le couvercle de fermeture (62) peut être ouvert et fermé après l'amarrage sur l'orifice de transfert rapide (56), le système d'arrimage, d'ouverture et de fermeture (36) comprenant un outil d'engagement (78), ainsi qu'un premier et un deuxième entraînement d'outil (80, 82) destiné au déplacement et à la rotation de l'outil d'engagement (78), et présentant également un raccord de fluide (76), l'outil d'engagement (78) pouvant être mis en prise avec le couvercle de fermeture (62) pour faire tourner et soulever le couvercle de fermeture (62) pour l'ouverture, et le raccord de fluide (76) servant à l'amenée de fluide de traitement dans le récipient (12) et/ou à l'évacuation de fluide de traitement à partir du récipient (12) à travers l'orifice de transfert rapide (56).

2. Dispositif de traitement selon la revendication 1, **caractérisé par** des moyens (38) destinés à l'orientation automatique du système d'arrimage, d'ouverture et de fermeture (36) dans une relation de position définie par rapport à l'orifice de transfert rapide (56).

3. Dispositif de traitement selon la revendication 2, **caractérisé en ce que** les moyens (38) d'orientation automatique comprennent des éléments d'orientation (54, 118) coopérant par paire qui sont raccordés de façon rigide au récipient (12) ou respectivement au système d'arrimage, d'ouverture et de fermeture (36).

4. Dispositif de traitement selon l'une des revendications précédentes, **caractérisé en ce que** le système d'arrimage, d'ouverture et de fermeture (36) est mobile par rapport au support (23).

5. Dispositif de traitement selon l'une des revendications précédentes, **caractérisé en ce que** le système d'arrimage, d'ouverture et de fermeture (36) comprend un capot (74) ouvert vers le bas et des moyens destinés à rendre un bord inférieur du capot (74) étanche vis-à-vis de l'orifice de transfert rapide (56) ou du récipient (12).

6. Dispositif de traitement selon la revendication 5, **caractérisé en ce que** le système d'arrimage, d'ouverture et de fermeture (36) présente un premier entraînement d'outil (80) destiné au soulèvement et à l'abaissement de l'outil d'engagement (78) par rapport au capot (74) et un deuxième entraînement d'outil (82) destiné à la rotation de l'outil d'engagement (78) par rapport au capot (74).

7. Dispositif de traitement selon la revendication 6, **caractérisé en ce que** le système d'arrimage, d'ouverture et de fermeture (36) comprend un disque (92) ou une plaque pouvant être déplacé(e) conjointement avec l'outil d'engagement (78) pour l'obturation étanche d'un creux (70) dans l'élément de fermeture (62) de l'orifice de transfert rapide (56).

8. Dispositif de traitement selon l'une des revendications précédentes, **caractérisé en ce que** le récipient de traitement et de transport (12) comprend des moyens (44) destinés à la mise en place détachable sur le support (22) pivotant du dispositif de traitement (10), une ouverture de remplissage et de vidage (48) unique pour les objets dirigée vers le haut dans une position de remplissage du récipient (12) et vers le bas dans une position de vidage du récipient (12) et un orifice de transfert rapide (56) mis en place devant l'ouverture de remplissage et de vidage (48) avec un couvercle de fermeture (62) qui peut être ouvert et fermé au moyen d'un système d'arrimage, d'ouverture et de fermeture (36) mis en place sur le support (22) et pivotant conjointement avec le support (22), **en ce que**, sur le récipient (12), à part l'ouverture de remplissage et de vidage (48), il n'est prévu qu'un unique raccord de fluide (46), et **en ce que** l'amenée et/ou l'évacuation de fluide de traitement à partir du dispositif de traitement (10) s'effectue d'une part à travers le raccord de fluide (46) ainsi que d'autre part à travers un raccord de fluide (76) du système d'arrimage, d'ouverture et de fermeture (36) amarré à l'orifice de transfert rapide (56) et l'orifice de transfert rapide (56).

9. Dispositif de traitement selon la revendication 8, **caractérisé en ce que** le raccord de fluide (46) est disposé à une extrémité du récipient (12) qui est opposée à l'ouverture de remplissage et de vidage (48).

10. Dispositif de traitement selon la revendication 8 ou 9, **caractérisé en ce que** le récipient (12) comprend des éléments d'orientation (54) à proximité de l'ouverture de remplissage et de vidage (48) et de l'orifice de transfert rapide (56), destinés à coopérer avec des éléments d'orientation (118) complémentaires du système d'arrimage, d'ouverture et de fermeture (36) du dispositif de traitement (10).

11. Dispositif de traitement selon l'une des revendications 8 à 10, **caractérisé par** un clapet percé pivotant (50) disposé dans l'ouverture de remplissage et de vidage (48) du récipient (12).

12. Procédé destiné au nettoyage et à la stérilisation ainsi qu'au transport d'objets relativement petits pour des utilisations pharmaceutiques, dans lequel une pluralité des objets sont introduits dans un récipient de traitement et/ou de transport (12) muni d'une unique ouverture de remplissage et de vidage (48) et d'un orifice de transfert rapide (56) mis en place devant l'ouverture de remplissage et de vidage (48), dans lequel le récipient (12) est mis en place ensuite de façon détachable sur un support(22), pouvant pivoter de façon motorisée, d'un dispositif de traitement (10), **caractérisé en ce que**, après la mise en place du récipient (12) sur le support (22), un couvercle de fermeture (62) de l'orifice de transfert rapide (56) est ouvert au moyen d'un système d'arrimage, d'ouverture et de fermeture (36) pivotant conjointement avec le support (22), et au moins un fluide de traitement est amené dans le récipient (12) ou évacué du récipient (12) à travers un raccord de fluide (76) du système d'arrimage, d'ouverture et de fermeture (36) ainsi qu'à travers l'orifice de transfert rapide (56) ouvert et évacué du récipient (12).

13. Procédé selon la revendication 12, **caractérisé en ce que** l'orifice de transfert rapide (56) est arrimé à un système d'arrimage, d'ouverture et de fermeture (36) du support (22) et est ouvert et refermé automatiquement par le système d'arrimage, d'ouverture et de fermeture (36).
